(19) Europäisches Patentamt
European Patent Office
Office européen des brevets

(11) EP 0 988 859 A1

(12) EUROPÄISCHE PATENTANMELDUNG

(43) Veröffentlichungstag:
29.03.2000 Patentblatt 2000/13

(51) Int. Cl.[7]: **A61K 9/38**, A23J 3/00, B01J 13/14, C08J 5/18, C08L 89/00

(21) Anmeldenummer: 99117095.2

(22) Anmeldetag: 31.08.1999

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: 03.09.1998 DE 19840069
04.09.1998 DE 19840489

(71) Anmelder:
**BASF AKTIENGESELLSCHAFT**
**67056 Ludwigshafen (DE)**

(72) Erfinder:
- **Friedrich, Thomas, Dr.**
  **64283 Darmstadt (DE)**
- **Bewert, Wolfgang, Dr.**
  **67227 Frankenthal (DE)**
- **Lüddecke, Erik, Dr.**
  **67112 Mutterstadt (DE)**
- **Klinger, Jürgen, Dr.**
  **Setagaya-ku, Tokio 158 (JP)**
- **Heger, Robert, Dr.**
  **69124 Heidelberg (DE)**

(54) **Wirkstoffzubereitungen sowie ein Verfahren zu deren Herstellung**

(57) Die vorliegende Erfindung betrifft Wirkstoffzubereitungen, bei denen ein oder mehrere Wirkstoffe von mindestens einer Schicht oder Teile einer Schicht aus einem Protein umgeben sind, das mit einem Enzym ausgewählt aus der Gruppe der Lipoxygenasen, Proteindisulfidisomerasen, Phenoloxidasen, Lysyloxidasen, Proteindisulfidreduktasen oder Sulfhydryloxidasen quervernetzt wurde.

Außerdem betrifft die Erfindung ein Verfahren zur Herstellung von Wirkstoffzubereitungen, in denen ein oder mehrere Wirkstoffe von mindestens einer Schicht umgeben sind, dadurch gekennzeichnet, daß mindestens eine der den oder die Wirkstoffe umgebenden Schichten oder Teile dieser Schichten aus einem Protein bestehen, das mit einem Enzym ausgewählt aus der Gruppe der Lipoxygenasen, Proteindisulfidisomerasen, Phenoloxidasen, Lysyloxidasen, Protieindisulfidreduktasen oder Sulfhydryloxidasen quervernetzt wurde sowie die Verwendung der genannten Enzyme zur Formulierung von Wirkstoffen.

Weiterhin betrifft die Erfindung Lebensmittel, Futtermittel oder pharmazeutische Zubereitungen enthaltend die erfindungsgemäße Wirkstoffzubereitung sowie das Enzym Lysyloxidase. Im Besonderen betrifft die Erfindung Trockenpulver, die Vitamine, Enzyme, Lebensmittel- und Futtermittelzusatzstoffe, wie z.B. Carotinoide enthalten. Außerdem können noch verschiedene Zusatzstoffe eingebettet sein.

EP 0 988 859 A1

## Beschreibung

Die vorliegende Erfindung betrifft Wirkstoffzubereitungen, bei denen ein oder mehrere Wirkstoffe von mindestens einer Schicht oder Teilen einer Schicht aus einem Protein umgeben sind, das mit einem Enzym ausgewählt aus der Gruppe der Lipoxygenasen, Proteindisulfidisomerasen, Phenoloxidasen, Lysyloxidasen, Proteindisulfidreduktasen, Tyrosinoxidasen oder Sulfhydryloxidasen quervernetzt wurde.

Außerdem betrifft die Erfindung ein Verfahren zur Herstellung von Wirkstoffzubereitungen, in denen ein oder mehrere Wirkstoffe von mindestens einer Schicht umgeben sind, dadurch gekennzeichnet, daß mindestens eine der den oder die Wirkstoffe umgebenden Schichten oder Teile dieser Schichten aus einem Protein bestehen, das mit einem Enzym ausgewählt aus der Gruppe der Lipoxygenasen, Proteindisulfidisomerasen, Phenoloxidasen, Lysyloxidasen, Proteindisulfidreduktasen, Tyrosinoxidasen oder Sulfhydryloxidasen quervernetzt wurde sowie die Verwendung der genannten Enzyme zur Formulierung von Wirkstoffen.

Weiterhin betrifft die Erfindung Lebensmittel, Futtermittel oder pharmazeutische Zubereitungen enthaltend die erfindungsgemäße Wirkstoffzubereitung sowie das Enzym Lysyloxidase. Im Besonderen betrifft die Erfindung Trockenpulver, die Vitamine, Enzyme, Lebensmittel- und Futtermittelzusatzstoffe, wie z.B. Carotinoide enthalten. Außerdem können gegebenenfalls noch verschiedene Zusatzstoffe eingebettet sein.

Pulverförmige Wirkstoffzubereitungen wie Vitamin- und Carotinoid-Präparate sind allgemein bekannt und werden in der pharmazeutischen Industrie sowie in der Nahrungs- und Futtermittelindustrie in großem Umfang verwendet. In der Literatur werden viele Verfahren zur Herstellung geeigneter Präparate beschrieben.

Zur Herstellung von pulverförmigen Zubereitungen, in denen oxidationsempfindliche Stoffe wie öllösliche Vitamine oder Carotinoide gegen oxidative Einflüsse geschützt werden sollen, werden verschiedene Herstellverfahren, insbesondere Sprühverfahren beschrieben.

In der deutschen Patentschrift 1 035319 wird beschrieben, wie eine Dispersion eines öligen Vitamins in einen hohen Überschuß von pulverförmiger Stärke mit einem geringen Feuchtegehalt (unter 8 %) versprüht wird. Durch das trockene Stärkepulver wird den versprühten Partikeln Wasser entzogen. Hierdurch erstarren sie, wobei eine große Menge der Stärke an der Oberfläche der Partikel haften bleibt. Außerdem muß der überschüssige Stärkeanteil abgetrennt und anschließend wieder dem Prozeß zugeführt werden.

In der schweizerischen Patentschrift 488 455 wird dargelegt, daß als Puderungsmittel ein Gemisch aus anorganischen Substanzen eingesetzt wird, das aus wasserabweisenden und wasserabsorbierenden Substanzen besteht. Hierdurch soll die Explosionsgefahr, die durch die trockene, feinverteilte Stärke besteht, vermieden werden.

Aus der schweizerischen Patentschrift 389 505 ist bekannt, daß die Dispersion des Wirkstoffs in ein gekühltes, gasförmiges Medium versprüht wird, in dem die versprühten Partikel durch Abkühlung erstarren. Für diesen Prozeß werden Fallhöhen von bis zu 15 m benötigt, außerdem müssen die Temperaturen deutlich unter Raumtemperatur gehalten werden.

Eine weitere Methode zur Verfestigung der versprühten Partikel kann auch durch Auffangen in einem Pulver, das aus Metallsalzen höherer Fettsäuren besteht, erfolgen. Dieses Verfahren wird in der schweizerischen Patentschrift 431 252 beschrieben.

Eine alternative Methode zur Herstellung von wirkstoffhaltigen, stabilen Zubereitungen wird in der europäischen Patentschrift EP-A-0 618 001 beschrieben. Hier erfolgt die Herstellung der kugelförmigen Partikel, die eine Einbettung des Wirkstoffs in einem Gemisch aus verschiedenen Trägersubstanzen darstellen, indem man zunächst durch kontrollierte Teilung Kügelchen aus einer primären Öl-in--Wasser-Emulsion bildet, die aus der Zugabe von Wirkstoffen, ölförmigen Stoffen, Proteinen und Wasser zum einem nicht mit Wasser mischbaren Lösungsmittel besteht, und anschließend die erhaltenen Kügelchen abtrennt. Für die Herstellung der Kügelchen ist ein spezielles Mischsystem erforderlich. Die hierbei erhaltenen Partikel werden anschließend mit einem Aldehyd, beispielsweise Acetaldehyd, Glutaraldehyd oder Glyoxal nachbehandelt, wodurch eine chemische Vernetzung, die sich auch in der Wasserunlöslichkeit des erhaltenen Materials ausdrückt, und damit eine zusätzliche Stabilisierung des Wirkstoffs erreicht wird.

In der amerikanischen Patentschrift 4 670 247 wird eine weitere Methode zur Herstellung von vernetzten Partikeln beschrieben. Hierzu wird zunächst eine Emulsion, die im wesentlichen aus einem öllöslichen Vitamin, einem Schutzkolloid wie z.B. Gelatine und einem reduzierenden Zucker besteht, durch einen Sprüh- und Trocknungsprozeß in pulverförmige Partikel überführt. Diese Partikel werden anschließend in einem thermischen Prozeß bei Temperaturen zwischen 105 und 180°C behandelt. In einer Maillard-Reaktion zwischen den Aminogruppen der Proteine und den Oxo-Gruppen der reduzierenden Zucker wird dabei eine Wasserunlöslichkeit der Trockenpulverpartikel erzielt, die durch eine Vernetzung der Matrixbestandteile erreicht wird.

In EP 782883 werden eßbare Mikrokapseln beschrieben, die eine Kapselwand enthalten, die durch Aussalzen des Proteins mit einem eßbaren Salz und Vernetzung der Kapselwand mit Transglutaminase hergestellt werden. Nachteilig bei dieser Methode ist, daß sie nicht breit anwendbar ist. So ist die Transglutaminase beispielsweise für Sprühformulierungen ungeeignet, da sie versprühte und getrocknete Wirkstofformulierungen nicht mehr richtig quervernetzten kann und so die Wirkstoffe während der Lagerung schon einem verstärkten Abbau ausgesetzt sind. Wartet man bis die

Quervernetzung durch die enzymatische Aktivität abgeschlossen ist, so lassen sich die Formulierungen nicht mehr versprühen.

Wenn oxidationsempfindliche Verbindungen, in diesem Falle insbesondere fettlösliche Vitamine und Carotinoide, mit Luft in Kontakt kommen, erfolgt eine Umsetzung mit Sauerstoff, die zu einem Wirkstoffverlust durch Umsetzung zu ungewünschten Verbindungen führt. Um diese Oxidation zu verhindern, kann man zum Beispiel bestimmte Zusätze den Zubereitungen hinzufügen, die durch Umsetzung mit reaktiven Gruppen der Proteine diese wiederum weniger durchlässig für Sauerstoff machen und dadurch den Wirkstoffen einen stabilisierenden Schutz verleihen. Dies kann beispielsweise dadurch geschehen, daß durch Umsetzung von Proteinen mit reduzierenden Zuckern im Sinne einer Maillard-Reaktion die Löslichkeit der Proteine in Wasser verhindert wird. Weiterhin kann durch Umsetzung der Proteine mit Aldehyden eine Vernetzung erreicht werden, die ebenfalls der Trägermatrix eine erhöhte Stabilität verleiht.

Diese Verfahren zeigen jedoch bestimmte Nachteile, die es wünschenswert erscheinen lassen, nach verbesserten Stabilisierungsverfahren zu suchen. So bedeutet die Anwendung einer Maillard-Reaktion zwischen einem Protein und reduzierenden Zuckern in jedem Falle eine thermische Belastung und damit zumindest in einem geringen Maße einen Abbau des Wirkstoffs. Außerdem neigen die Produkte zu einer bräunlichen Färbung. Ein Verfahren zur Formulierung von Wirkstoffen über Maillard-Reaktion ist beispielsweise der Patentanmeldung EP-A-0 547 422 zu entnehmen.

Von Nachteil bei der Verwendung von chemischen Agentien wie Aldehyden oder Säuren wie Tanninsäure als Vernetzungsmittel ist, daß zur Vernetzung hochreaktive und gesundheitlich nicht unbedenkliche Zusatzstoffe eingesetzt werden. Die Produkte, die nach diesen Verfahren hergestellt werden, finden beim Verbraucher nur bedingt unumschränkte Akzeptanz.

Es war daher die Aufgabe der Erfindung ein leichtes, kostengünstiges, breit anwendbares Verfahren zur Formulierung von Wirkstoffen zu entwickeln, das die oben genannten Nachteile nicht aufweist.

Diese Aufgabe wurde durch das erfindungsgemäße Verfahren zur Herstellung von Wirkstoffzubereitungen, in denen ein oder mehrere Wirkstoffe von mindestens einer Schicht umgeben sind, dadurch gekennzeichnet, daß mindestens eine der den oder die Wirkstoffe umgebenden Schichten oder Teile dieser Schichten aus einem Protein bestehen, das mit einem Enzym ausgewählt aus der Gruppe der Lipoxygenasen, Proteindisulfidisomerasen, Phenoloxidasen und Peroxidasen, Lysyloxidasen, Proteindisulfidreduktasen, Tyrosinoxidasen oder Sulfhydryloxidasen quervernetzt wurde, gelöst.

Die chemische Funktion dieser Enzyme im Stoffwechsel bzw. ihre chemische Reaktion ist beispielsweise den Schriften von Green et al. (Biochem. J. 1983, 211, 481 - 493), Kagan et al. (Am. J. Respir. Cell Mol. Biol., 5, 1991, 206 - 210), Haywood et al. (Biochem. J., 1981, 199, 187 - 201) oder Matheis et al. (J. Food Biochem. 11, 1987, 309 - 327) zu entnehmen

Durch die vorteilhafte enzymatischen Vernetzung im erfindungsgemäßen Verfahren wird sowohl eine thermische Belastung der Wirkstoffe wie bei der thermischen Vernetzung der Protein/Zuckerschicht und ein damit verbundenes Bräunen der Wirkstoffformulierungen über die Maillardreaktion sowie die Verwendung reaktiver chemischer Substanzen wie Aldehyde vermieden. Die Maillardreaktion bei der thermischen Vernetzung führt zu einer Verknüpfung von reduzierenden Zuckern mit den freien Aminogruppen der Proteine oder sonstigen freien Aminogruppen und damit zu einer Stabilisierung der Wirkstofformulierungen. Bei der Verwendung von chemischen Verbindungen zur Vernetzung der Proteine bilden sich Brücken zwischen den Aldehyden in der Regel den Dialdehyden und den Aminogruppen der Proteine oder sonstigen Aminogruppen aus. Im erfindungsgemäßen Verfahren werden die Proteine direkt beispielsweise über in den Proteinen oder Lipoproteinen vorhandenen Fettsäureresten, über Cyctein-Cysteinbrücken (= Cystinbrücken), über Michaeladditionsprodukte oder über die Bildung von Aldehyden aus Lysin mit anschließender Vernetzung über freie Aminogruppen vernetzt.

Dies führt zu den neuen erfindungsgernäßen Wirkstoffzubereitungen enthaltend mindestens eine einen oder mehrere Wirkstoffe umgebende Schicht oder Teile einer Schicht aus einem Protein, das mit einem Enzym ausgewählt aus der Gruppe der Lipoxygenasen, Proteindisulfidisomerasen, Phenoloxidasen und Peroxidasen, Lysyloxidasen, Proteindisulfidreduktasen, Tyrosinoxidasen oder Sulfhydryloxidasen quervernetzt wurde. Diese erfindungsgemäßen Wirkstoffzubereitungen weisen bei einer guten Stabilität keine bräunliche Färbung auf und enthalten keine chemischen Vernetzer.

Als Wirkstoffe im erfindungsgemäßen Verfahren oder in den erfindungsgemäßen Wirkstoffzubereitungen sind prinzipiell alle in der Pharmazie, Human- oder Tierernährung verwendeten Wirkstoffe geeignet. Bevorzugte Wirkstoffe sind Vitamine, Enzyme. Lebensmittel- und Futtermittelzusatzstoffe.

Unter Enzymen im erfindungsgemäßen Verfahren oder in den Wirkstoffzubereitungen sind als Wirkstoff Enzyme wie z.B. Hydrolasen wie Amidasen, Proteasen, Lipasen, Esterasen, Phospholipasen, β-Glucosidasen, Amylasen, Nitrilasen, Mannanasen, Phytasen oder Xylanasen, Transferasen wie Methyltransferasen oder Amino-transferasen, Oxidoreduktasen wie Glucoseoxidase oder Isomerasen beispielhaft zu verstehen.

Insbesondere sind hydrophobe Wirkstoffe bevorzugt, besonders bevorzugt solche, die leicht oxidierbar sind. Solche sind die Vitamine der Gruppen A, D, E und K sowie deren Mischungen speziell den Mischungen mit Carotinoiden und/oder Xanthophyllen. Sie können im Rahmen der Erfindung in Form von Vitaminlösungen in Ölen, als Provitamine sowie als reine Vitamine natürlichen oder synthetischen Ursprungs eingesetzt werden. Von besonderem Interesse sind Präparate von Vitamin $D_3$, Vitamin $K_1$, Vitamin A und dessen Derivaten, insbesondere von Vitamin-A-Acetat, Vitamin-A-Palmitat und Vitamin-A-Propionat sowie deren Mischungen. Auch die verschiedenen Vitamin E-Derivate wie Vitamin E-Acetat, Vitamin E-Palmitat sind von Interesse. Bevorzugte Lebensmittel-und Futtermittelzusatzstoffe sind Carotine, Xanthophylle und Carotinoide wie β-Carotin und wie z.B.: Astaxanthin, Astacin, Bixin, Norbixin, Capsorubin, Violaxanthin, Rubixanthin, Rhodoxanthin, Apo-8' -carotinsäureethylester, Neurosporaxanthin, Citranaxanthin, Canthaxanthin, Zeaxanthin, β-Apo-4'-carotinal, β-Apo-8'-carotinal, β-Apo-12'-carotinal, β-Apo-8'-carotinsäure, Lutein, Capsanthin, Lycopin sowie Ester von hydroxy- und carboxyhaltigen Vertretern dieser Gruppe, z.B. die niederen Alkylester wie Methyl- oder Ethylester oder deren Mischungen.

Die Anteile an den Wirkstoffen betragen in den erfindungsgemäßen Wirkstoffzubereitungen im allgemeinen etwa 1 bis 75 Gew.-%, vorzugsweise 5 bis 50 Gew.-%, besonders bevorzugt 10 bis 35 Gew.-%, bezogen auf die Trokkenmasse der Pulver.

Die Anteile an Vitaminen oder Carotinoiden betragen im allgemeinen etwa 5 bis 50 Gew.-%, vorzugsweise 10 bis 35 Gew.-%, bezogen auf die Trockenmasse der Pulver.

Bevorzugt sind erfindungsgemäße Verfahren, bei denen eine wirkstoffhaltige Emulsion oder Dispersion in eine mit hydrophobierter Kieselsäure beladene Atmosphäre, die gegebenenfalls inertisiert sein kann, versprüht wird. Die Atmosphäre kann vorteilhaft auch mit einem anderen Puderungsmittel wie einem Mittel auf Basis eines Kohlenhydrats wie Stärke oder modifizierter Stärke beispielsweise Maisstärke beladen sein.

Außerdem sind Verfahren bevorzugt, bei denen nach dem Herstellen der Wirkstoffzubereitung beispielsweise über Versprühen getrocknet wird. Dabei wird bevorzugt bis zu einer Restfeuchte unter 10 Gew.-%, bevorzugt unter 6 Gew.-% getrocknet. Auch geringere Restfeuchten können eingestellt werden.

Die Temperatur des erfindungsgemäßen Verfahrens wird im Wesentlichen unter 80°C, bevorzugt unter 60°C, gehalten.

Die Erfindung betrifft außerdem Wirkstoffzubereitungen erhältlich nach einem erfindungsgemäßen Verfahren, und solche, die als zusätzliches Merkmal den 0,025-fachen bis 4-fachen Gewichtsanteil bezüglich Wirkstoff an Trennmittel oder Trennmittelgemischen enthalten, sowie Lebensmittel oder Futtermittel enthaltend eine solche Wirkstoffzubereitung.

Bevorzugte Trennmittel sind hydrophobierte Kieselsäuren, Maisstärke, durch chemische Behandlung hydrophobierte Maisstärke, Metallsalze höherer Fettsäuren und andere pflanzliche Stärken oder Mischungen dieser Trennmittel. Besonders bevorzugt sind Mischungen aus mindestens einem dieser Trennmittel mit weiteren Hilfsstoffen wie anorganischen Verbindungen, die als Gleitmittel wirken, wie zum Beispiel Neusilin® oder Zeolex®.

Im Fall von hydrophober Kieselsäure liegt der Anteil bezüglich Wirkstoff bevorzugt in einem Bereich zwischen 0,025 und 0,4, besonders bevorzugt zwischen 0,05 und 0,2. Bei Maisstärke liegt das entsprechende Verhältnis bevorzugt zwischen 0,25 und 2, besonders bevorzugt zwischen 0,5 und 1,5.

Die erfindungsgemäßen Wirkstoffzubereitungen sind erhältlich durch Herstellung einer Dispersion enthaltend im wesentlichen diese Wirkstoffe, ein Protein und weitere Träger- und Füllstoffe z.B. aus der Gruppe der Kohlenhydrate und/oder natürliche oder chemisch modifizierte Stärken. Sie kann noch weitere Zusatzstoffe wie Stabilisatoren oder Emulgierhilfsmittel enthalten. Außerdem enthält sie ein Enzym, das in der Lage ist, auf verschiedene Weise Proteinmoleküle miteinander zu verknüpfen. Die hierdurch erreichte Vernetzung verleiht dem Protein und damit auch der Matrix, in dem die Wirkstoffe eingebettet sind, eine verminderte Wasserlöslichkeit und somit eine erhöhte Stabilität.

Als Protein im erfindungsgemäßen Verfahren können prinzipiell alle Proteine verwendet werden. Bevorzugte vernetzbare Proteine sind aus wirtschaftlichen Gründen Gelatine, Kasein, Soja-Protein, Weizenproteine, Mais-Protein und Kollagen.

Bevorzugte vernetzbare Proteine sind alle Formen von pflanzlichen oder tierischen Proteine wie Gelatine, z.B. Knochengelatine, Rindergelatine, Fischgelatine, Milchproteine, wie z.B. Kasein, Sojaproteine, Weizenproteine wie Gluten, Maisproteine und Kollagene, besonders bevorzugte Proteine sind Gelatine, Milchproteine und Sojaproteine. Unter Gelatine im erfindungsgemäßen Verfahren sind alle Formen von Gelatine zu verstehen, egal, ob es sich um natürliche Gelatinen handelt oder um chemisch modifizierte Derivate.

Für die Herstellung der Dispersion wird bei dem erfindungsgemäßen Verfahren vorteilhaft Gelatine des A- und B-Typs in einem weiten Bloombereich eingesetzt. Mit besonderem Vorteil arbeitet man mit Gelatine mit einem Bloomwert von etwa 50 bis etwa 250.

Die Gelatine verwendet man erfindungsgemäß in Mengen von 10 bis 50 Gew.-%, vorzugsweise 15 bis 40 Gew.-%, insbesondere 20 bis 35 Gew.-%, bezogen auf die Trockenmasse der Wirkstoffzubereitung.

Vorteilhaft werden den Proteinen zur mechanischen Stabilisierung Weichmacher wie Zucker und/oder Zuckeralkohole wie Saccharose, Glucose, Sorbit, Sorbose, Mannit oder Polyole wie Glycerin zugesetzt.

Die erfindungsgemäßen vernetzenden Enzyme sind ausgewählt aus der Gruppe der Lipoxygenasen, Proteindisulfidisomerasen (bevorzugt E.C.-Klasse 5.3.4), Phenoloxidasen (bevorzugt E.C.-Klasse 1.14.) und Peroxidasen (

bevorzugt E.C.-Klasse 1.11), Lysyloxidasen (bevorzugt E.C.-Klasse 1.4.3), Proteindisulfidreduktasen (bevorzugt E.C.-Klasse 1.6.4), Tyrosinoxidasen (bevorzugt E.C.-Klasse 1.14) oder Sulfhydryloxidasen (bevorzugt E.C.-Klasse 1.8.). Diese können tierischen, pflanzlichen oder mikrobiellen Ursprungs sein. Bevorzugt sind sie mikrobiellen Ursprungs, das heißt aus Eukaryonten wie Pilzen oder Hefen oder Prokaryonten wie grampositiven oder gram-negativen Bakterien oder Archaebakterien. Bevorzugt werden als Enzym die Lysyloxidasen verschiedenen Ursprungs verwendet. Besonders bevorzugt werden die Lysyloxidasen aus Hefen wie aus den Gattungen Candida, Hansenula, Pichia, Sporobolomyces, Sporopachydermia, oder Trigonopsis verwendet. Ganz besonders bevorzugt sind Lysyloxidasen aus den Gattungen und Arten Candida nagoyaensis, Candida nemodendra, Candida boidinii, Candida lipolytica, Candida stealolytica, Candida tropicalis, Candida utilis, Hansenula minuta, Hansenula polymorpha, Pichia pinus, Pichia pastoris, Sporobolomyces albo-rubescens, Sporopachydermia cereana oder Trigonopsis variabilis. Herausragend geeignet für das erfindungsgemäße Verfahren ist die Lysyloxidase aus der Gattung und Art Pichia pastoris.

Die erfindungsgemäße Lysyloxidase von Pichia patoris läßt sich aus der Zellmaße von Pichia pastoris Zellen über einen Zellaufschluß, der mit üblichen Methoden durchgeführt wurde, einer Ionenaustauschchromatographie mit einer anschließenden Molekularsiebchromatographie und abschließender nochmaliger Ionenaustauschchromatographie aufreinigen. Mit diesem Reinigungsschema konnte die Lysyloxidase mit einer Reinheit von über 90 %, bevorzugt von über 95 %, besonders bevorzugt von über 99 % dargestellt werden.

Die aufgereinigte Lysyloxidase wurde einer Proteinsequenzierung nach Ednan unterzogen. Dabei wurde das N-terminale Ende sowie verschiedene Peptide, die in einem Verdau mit Trypsin erhalten wurden, bestimmt (siehe Beispiel 5).

Die Erfindung betrifft damit ein isoliertes Protein, das mindestens eine der folgenden Sequenzen enthält

eine aminoterminale Sequenz

G(S)(C)Q(C)KTNEKVNIEAPKPNI(C)DT(L)(S)

oder Teilsequenzen, die Peptiden des Proteins entsprechen, die nach einem Verdau mit Trypsin erhalten wurden:

EYP(C)APGVVYNTK
GGTYSTVTQNPTLNR
DYNIMPGGGXVHR
ATGGTYSTVXAQN
APETENNAR
GPL(P)VNE(E)TTIEPLSFYNT
IYELSLQELIAEYGSDDPNNQHTFYSDI
DNVDDLSCTIIQR
VAPETENCAR
NVDVEYPCAPGVVYNTK
GYPNAEY(S)LDF/ER

und das die ε-Aminogruppen des Lysins speziell in einem Protein zu Aldehydgruppen oxidieren kann. Die Buchstaben und Zeichen in den oben genannten Sequenzen haben folgende Bedeutung: X ist eine unbekannte Aminosäure, die an dieser Position nicht identifiziert werden konnte, die Klammern bedeuten nicht eindeutig ermittelbare Aminosäuren, wobei die angegebenen Aminosäure, die wahrscheinlich richtige Aminosäure ist, der Schrägstrich bedeutet Aminosäurealternativen, wobei die Aminosäure vor dem Schrägstrich oder die nach dem Schrägstrich an der Position richtig sein kann. Durch die Oxidation der ε-Aminogruppen kommt es schließlich zur Quervernetzung von Proteinen, wobei Bereiche desselben Proteins miteinander vernetzt werden können oder verschiedener Proteine.

Die Lysyloxidase (= PROTEIN-LYSINE 6-OXIDASE, EC 1.4.3.13 oder LYSYL OXIDASE) speziell die Lysyloxidase von Pichia pastoris ermöglicht die enzymatische Quervernetzung von Proteinen, wobei als Protein vorteilhaft Gelatine verwendet wird. Dabei werden die ε-Aminogruppen der Lysine zu Aldehydgruppen oxidiert. Diese Aldehyde können dann unter Bildung Schiff'scher Basen mit den Aminogruppen anderen Lysine oder anderen freien Aminogruppen beispielsweise Aminozuckern reagieren. Ein besonderer technischer Vorteil für den Anwender ist die zeitliche Trennung innerhalb der gesamten Reaktionssequenz, das heißt, die Bildung der Aldehyde aus den ε-Aminogruppen der Lysine und die Reaktion der Aminogruppen mit den gebildeten Aldehydgruppen ist zeitlich getrennt. Dies bietet gegenüber beispielsweise der enzymatischen Quervernetzung mit Hilfe der Transglutaminase einen wesentlichen Fortschritt, da bei dieser Reaktion immer sofort die Vernetzung erfolgt und damit keine zeitliche Trennung in der Enzymreaktion und der Vernetzung möglich ist. Diese zeitliche Trennung ermöglicht die vorteilhafte Verwendung der enzymatischen Reaktion für Verfahren wie zum Beispiel die Herstellung von Wirkstoffzubereitungen über Sprühtrocknung oder über das sogenannte Mikronisierungsverfahren (siehe EP-A-0 065193). Ein weiterer Vorteil ist die Unbedenklichkeit der

gebildeten Moleküle, da sie ein natürlicher Bestandteil des Bindegewebes sind (Abbildung 1 und 2). Sie werden dort von einer Gewebe-Lysinoxidase erzeugt.

Abbildung 1: Bildung einer Aldolquerbrücke aus zwei Lysinseitenketten

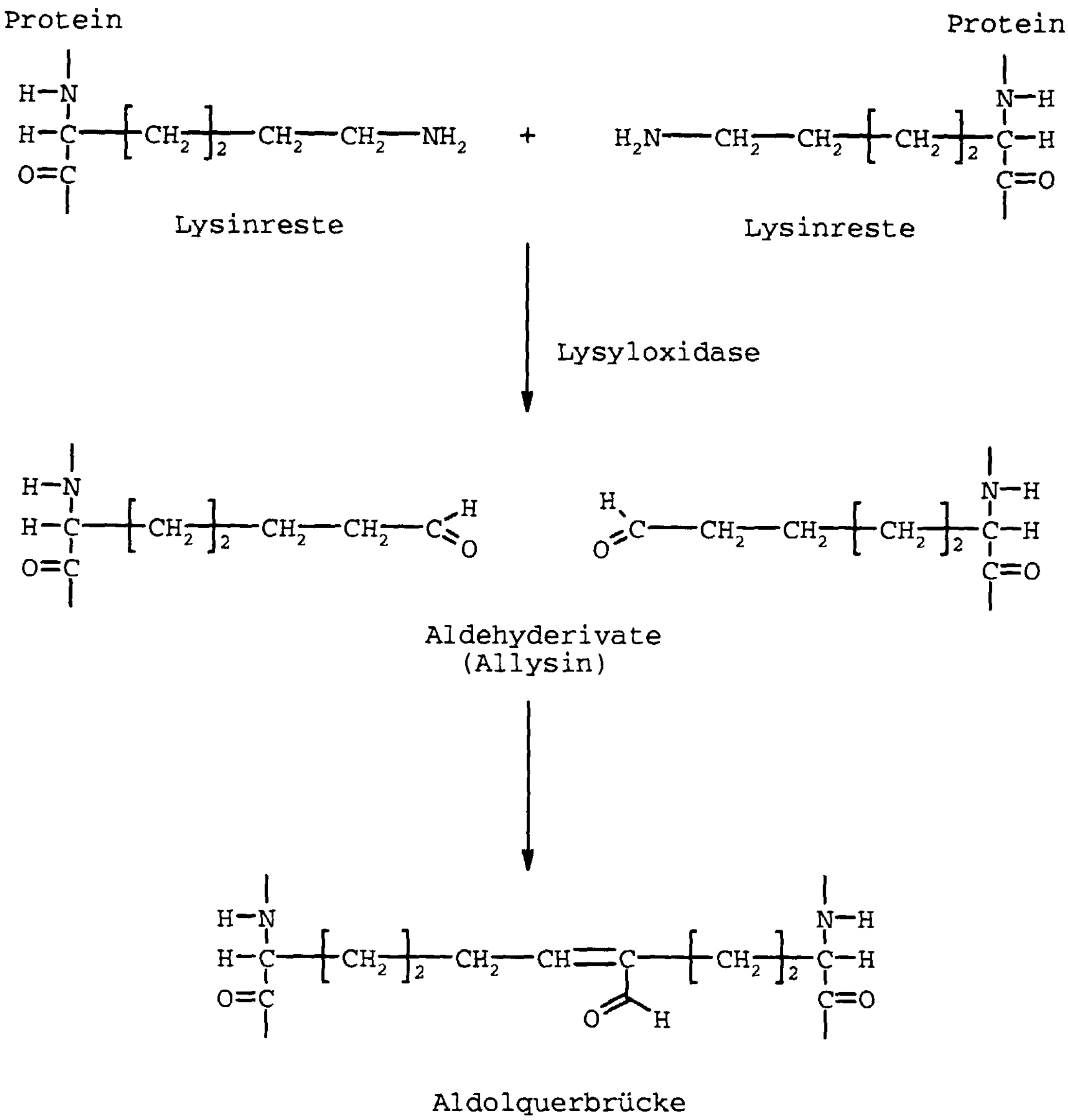


Lysinreste

Lysinreste

Aldehyderivate (Allysin)

Aldolquerbrücke

Die genannten Enzyme können in Form der gereinigten Enzyme oder in Form von Rohextrakten aus natürlichen Quellen wie aus Eukaryonten wie Pilzen, Hefen, tierischen oder pflanzlichen Zellen oder Prokaryonten wie grampositiven, gram-negativen Bakterien oder Archaebakterien verwendet werden. Auch ganze Organismen oder Zellen können für das erfindungsgemäße Verfahren verwendet werden, soweit die Enzyme ins extrazelluläre Milieu sezerniert werden oder die Zellen permeabilisiert wurden. Bevorzugt wird das erfindungsgemäße Verfahren mit gereinigten Enzymen oder, soweit keine unerwünschten Nebenaktivitäten vorhanden sind, mit Rohextrakten durchgeführt.

Die für die Quervernetzung der Proteine benötigten Enzymmengen bzw. -aktivitäten lassen sich in herkömmlicher Weise in dem Fachmann bekannter Art durch einfache Vorversuche bestimmen. Üblicherweise werden die Enzyme in einer Menge zugesetzt, daß 20 bis 100 % der quervernetzbaren Gruppen beispielsweise der Lysinreste im Protein vernetzt werden. Vorteilhafterweise werden 0,001 bis 1000 Units Enzym pro Gramm Protein, vorzugsweise 0,01 bis 100 Units Enzym pro Gramm Protein, besonders bevorzugt 0,1 bis 10 Units pro Gramm Protein zugesetzt.

Die Wirkstoffzubereitungen können einen oder mehrere Wirkstoffe enthalten, wobei unterschiedliche Wirkstoffklassen wie Vitamine oder Carotinoide oder mehrere Wirkstoffe einer Wirkstoffklasse beispielsweise mehrere verschiedene Carotinoide oder Vitamine oder deren Mischungen in den Zubereitungen enthalten sein können.

Die Wirkstoffe in den Zubereitungen können von einer oder mehreren Schichten umgeben sein. Diese Schichten können aus einer oder mehreren erfindungsgemäß quervernetzten Proteinschichten oder aus einer oder mehreren erfindungsgemäß quervernetzten Proteinschichten und mindestens einer chemisch quervernetzten Proteinschicht und/oder weiteren Schichten aus natürlichen oder künstlichen Polymeren bestehen. Die nach dem erfindungsgemäßen Verfahren quervernetzten Proteine können auch Teile mindestens einer den oder die Wirkstoffe umgebenden Schicht sein.

Unter natürlichen und/oder künstlichen Polymeren sind prinzipiell alle Polymere denkbar, die für die Formulierung von Wirkstoffen geeignet sind. Als natürlichen Proteine seihen beispielsweise Polymere wie Guar Gum, Alginate, Carrageenan, Pektine oder Polymere auf Basis von Mannose und/oder Galaktose genannt. Als künstliche Proteine seinen beispielsweise Polymere auf Basis von Acrylsäure, Methacrylsäure, Acrylaten, Methacylaten oder Mischungen aus diesen Monomeren wie beispielsweise Eudragit L30D-55 (Methacrylsäure : Ethylacrylat, 1 : 1) oder Eudragit S (Methacrylsäure Methylmethacrylat, 1 2). Auch andere Monomere können in den Polymeren enthalten sein beipielsweise Monomere, die eine positive Ladung mitbringen wie Trimethlyammonioethylmethacrylat, Polylysin oder Polyallylamin. Je nach verwendetem Polymer oder Polymergemisch lassen sich mit Hilfe der erfindungsgemäßen Schichten herstellen, die es vorteilhaft ermöglichen den oder die Wirkstoffe so zu formulieren, daß sie gezielt am Wirkort freigesetzt werden können. So lassen sich Schichten erzeugen die beispielsweise Magensaft resistent sind, die sich im Darm auflösen, die sich gezielt im Magen auflösen, die durch das Pansenmileu nicht aufgelöst werden oder die bei Verbringung in den Körper den Wirkstoff erst nach einiger Zeit freigeben. In Futter- oder Nahrungsmitteln kann der Wirkstoff so formuliert werden, das er erst nach Aufnahme der Wirkstoffzubereitung in den Körper freigesetzt wird.

Vorteilhaft wird der Wirkstoff oder die Wirkstoffe von einer oder mehreren über das erfindungsgemäße Verfahren quervernetzten Proteinschichten umgeben.

Die die Wirkstoffzubereitungen enthaltenen Emulsionen werden vorteilhaft unter Verwendung von hydrophoben Trennmitteln wie hydrophobe Kieselsäure, natürliche Stärke, wie z.B. Maisstärke, hydrophobierte Stärkederivate, wie z.B. hydrophobierte Maisstärke, Salzen von langkettigen Fettsäuren oder Gemischen aus diesen Stoffen versprüht. Das Trennmittel kann aber auch in der Sprühkammer z.B. als hydrophobe Kieselsäurepartikel in Luft oder Inertgas (z.B. Stickstoff) vorgelegt werden. Anschließend erfolgt die Trocknung der versprühten Partikel gegebenenfalls nach Abtrennung der Trennmittel, gegebenenfalls unter leichter Erwärmung bis 80°C, bevorzugt bis 60°C, besonders bevorzugt bei Raumtemperatur, durch Behandlung in einem Luft- oder Schutzgasstrom.

Zusätzlich zu den obligatorischen Bestandteilen werden der Dispersion mit Vorteil noch andere, bei der Herstellung von Wirkstofftrockenpulvern übliche Verbindungen, zugefügt.

Abbildung 2: Reaktion der Lysinreste mit Allysin zur Schiff'schen Base und weitere Reaktion zu Lysinonorleucin, Desmosin und Isodesmosin

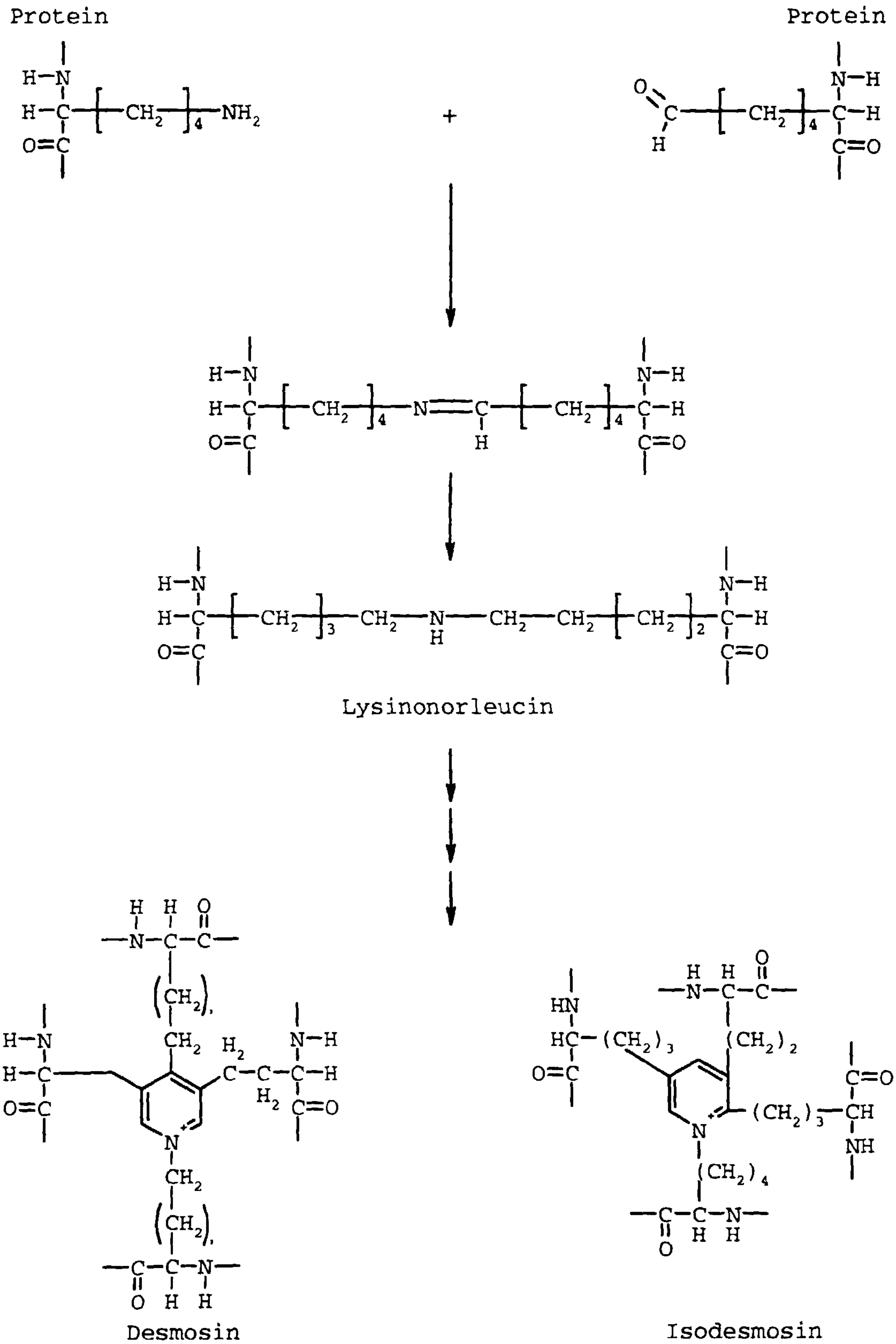

Lysinonorleucin

Desmosin

Isodesmosin

Von besonderer Bedeutung für einen Einsatz der Trockenpulver als Futtermittelzusatz ist bei oxidationsempfindlichen Wirkstoffen ein Zusatz von Antioxidantien, wie Ethoxyquin, butyliertes Hydroxytoluol (BHT), butyliertes Hydroxyanisol (BHA) oder Tocopherol, sowie Stabilisatoren, wie Ascorbylpalmitat, Mono- und Diglyceride, Ester von Monoglyceriden mit Essigsäure, Zitronensäure, Milchsäure, Diacetylweinsäure, Polyglycerinfettsäureester, Sorbitanfettsäureester, Propylenglykolfettsäureester, Stearoyl-2-Lactylat, Phosphorsäure oder Phytinsäure und deren Alkali- oder Erdalkalisalze, oder aber Komplexbildner, wie Ethylendiamintetraessigsäure (EDTA) oder Nitrilotriessigsäure (NTA).

Häufig werden der Emulsion aber auch Feuchthaltemittel, wie Glycerin, Sorbitol, Polyvinylpyrrolidon oder Polyethylenglykole oder auch zusätzliche Emulgatoren, wie Lecithin, zugefügt.

Darüberhinaus haben sich Zusätze wie Kohlenhydrate wie Zucker, Stärke oder Stärkederivate insbesondere Maisstärke oder Maltodextrin oder Dickungsmittel, wie Gummiarabicum, Guargummi, Traganth, AgarAgar, Carrageenan, Locust-Bean-Gum, Alginate, Pektine, Xanthan, Curdlan und bestimmte abgebaute Stärken zur Einstellung der Viskosität der Emulsion als nützlich erwiesen.

Bezüglich näherer Details über Bedeutung, Art und Menge solcher Zusätze verweisen wir auf entsprechende Fachliteratur, beispielsweise auf die obengenannte Monographie "Fat-soluble Vitamins", Vol. 9, insbesondere Seiten 128 bis 133.

Durch diese Zusätze werden die Wirkstoffzubereitungen vorteilhaft stabilisiert und an die besonderen Darreichungsbedingungen angepaßt.

Zur Durchführung des erfindungsgemäßen Verfahrens geht man beispielsweise so vor, daß man zur Herstellung der die Wirkstoffe enthaltenden Dispersion Gelatine in heißem Wasser (von 50 bis 70°C) zur Lösung bringt, zu dieser Lösung die Zucker, die Aminoverbindungen, die Vitamine und/oder Carotinoide, Stabilisatoren und die anderen üblichen Zusatzstoffe sowie ggf. noch zusätzlich Wasser addiert und das Ganze durch kräftiges Rühren bei erhöhter Temperatur dispergiert. Für die im letzten Verarbeitungsschritt erfolgende enzymatische Vernetzung des Pulvers sollte die fertige Dispersion in einem pH-Bereich von 4 bis 10 vorliegen, welcher durch Zugabe von Basen, wie NaOH, KOH, $Ca(OH)_2$, MgO, Soda oder $NH_4OH$ oder durch Zugabe von basisch reagierenden Salzen wie Natriumacetat oder $Na_2HPO_4$ eingestellt werden kann. Die so hergestellte Dispersion kann nach üblichen Methoden in ein trockenes Pulver überführt werden beispielsweise durch Sprühtrocknung, Sprühgranulation oder sonstige übliche Trocknungsmethoden.

Weitere Ausführungsformen des erfindungsgemäßen Verfahrens sind beispielsweise die Verwendung der enzymatischen Quervernetzung bei den unterschiedlichen Mikronisierungsverfahren.

In EP-B-0 065 193 wird beispielsweise ein Verfahren zur Herstellung von sehr feinverteilten freifließenden Pulvern der Carotinoide beschrieben, wobei das Carotinoid in einem flüchtigen, mit Wasser mischbaren organischem Lösungsmittel bei Temperaturen zwischen 50°C und 200°C, gegebenenfalls unter erhöhtem Druck, innerhalb einer Zeit von weniger als 10 Sekunden gelöst wird und anschließend die so erhaltene molekulardisperse Lösung sofort durch schnelles Mischen mit einer wäßrigen Lösung eines quellbaren Kolloids bei Temperaturen zwischen 0°C und 50°C der Wirkstoff in kolloiddisperser Form ausfällt. Die Dispersion wird anschließend vom Lösungsmittel und dem Dispergiermedium in üblicher Weise befreit. Als quellbare Kolloide werden Proteine verwendet. Diese können durch Zugabe der oben genannten Enzyme im erfindungsgemäßen Verfahren quervernetzt werden. Vorteilhafterweise werden die Enzyme der wäßrigen Mikronisatlösung vor dem Trocknungsschritt zugesetzt, damit genügend reaktive Gruppen für die Vernetzung hergestellt werden, bevor die Trocknung erfolgt. Weitere Angaben zum Verfahren sind EP-B-0 065 193 zu entnehmen.

EP-B-0 410 236 beschreibt ein weiteres Verfahren zur Herstellung einer kolloid-dispersen Carotinoidpräparation, wobei eine Suspension eines Carotinoides in einem hochsiedenden Oel während einer Zeitdauer von höchstens 30 Sekunden mit überhitztem Wasserdampf in Kontakt gebracht wird. Anstelle des Oels für das Lösen der Carotinoide können auch organische Lösungsmittel wie Chloroform, Methylenchlorid, Tetrachlorkohlenstoff oder Trichlorethylen verwendet werden (siehe DE/OS 1211911). Anschließend wird das erhaltene Gemisch mit einer wäßrigen Lösung eines Schutzkolloids emulgiert und die Emulsion danach versprüht und getrocknet. Auch hier werden als Schutzkolloide Proteine verwendet, die im erfindungsgemäßen Verfahren über die Zugabe der genannten Enzyme vor dem Vermischen und Versprühen quervernetzt werden können. Weitere Angaben zum Verfahren sind EP-B-0 410 236 zu entnehmen.

EP-B-0 498 824 beschreibt ein zusätzliches vorteilhaftes Verfahren zur Herstellung von mikrodispersen Wirkstoffzubereitungen. Dabei wird als Wirkstoff ein Carotinoid in Gegenwart eines Schutzkolloides beispielsweise eines Proteins wie Gelatine gemahlen und die gebildete Suspension anschließend getrocknet.

Durch Zusatz der im erfindungsgemäßen Verfahren verwendeten Enzyme lassen sich die Proteine vorteilhaft vernetzen und die Wirkstoffzubereitung stabilisieren. Nähre Angaben zum Herstellverfahren sind der genannten Patentschrift und WO 94/19411 zu entnehmen.

Die anschließende Weiterverarbeitung der Dispersion zu den erfindungsgemäßen Pulvern kann nach allen literaturbekannten Verfahren erfolgen.

Wegen der gewünschten Kornverteilung der Pulver (0,1 bis 0,6 mm Durchmesser) werden solche Verfahren bevorzugt, bei denen Vorkehrungen getroffen werden, daß die gelierten Tröpfchen der Dispersion voneinander getrennt bleiben, bis sich ihre Form stabilisiert hat.

Genannt seien beispielsweise das Verfahren gemäß EP-B1-74050, bei dem die Dispersion in hydrophobe Kieselsäure oder ein Metallsalz einer höheren Fettsäure versprüht wird oder aber das Verfahren gemäß EP-B1 285 682, bei dem die Dispersion in Stärkepulver versprüht wird. Es hat sich erwiesen, daß insbesondere bei Verfahren, die für Zubereitungen mit Gelatine-Gehalten unter 35 Gew.-% eingesetzt werden, die Versprühung mit hydrophobierten Kieselsäuren als Pudermittel besonders vorteilhaft durchführbar ist.

Die nach den beschriebenen Verfahren erzeugten Pulver besitzen nach dem Trocknen einen Wassergehalt von weniger als 10 %, normalerweise weniger als 6 %. Die so erhaltenen pulverförmigen Präparate bestehen aus Teilchen mit gut ausgebildeter Oberfläche. Sie lösen sich in warmem Wasser von ca. 40°C rasch zu einer milchigen Dispersion.

Die erfindungsgemäßen Wirkstoffzubereitungen eignen sich vorteilhaft zur Herstellung pharmazeutischer Zubereitungen, sowie zur Herstellung von Lebensmittel und Futtermittel.

Beispiele

Beispiel 1

Herstellung der Lysyloxidase mit Pichia pastoris - Lu 583 -.

Der Stamm wurde auf YM-Agar kultiviert:

| | | |
|---|---|---|
| 3 g/l | Malt Extract | Difco |
| 5 g/l | Pepton | Difco |
| 3 g/l | Yeast Extract | Difco |
| 20 g/l | Agar | Difco |

Die Platten wurden bei 28°C für 48 h bebrütet und sind danach bei 45°C für mindestens 4 Wochen haltbar.

Vorkultur und Fermentationsmedium:

10 g/l Glucose (30 min. bei 121°C autoklavieren)

separat ansetzen und 30 min bei 121°C autoklavieren:

0.2 g/l Magnesiumsulfat x 7 $H_2O$
3.0 g/l Kaliumdihydrogenpohsphat
0.2 ml/l Salzlösung nach Vishniac & Santer Rezeptur der Salzlösung. Nach Vishniac & Santer*

separat ansetzen und sterilfiltrieren:

10 ml/l Vitaminlösung nach Lodder Rezeptur der Vitaminlösung nach Lodder**

Die Mediumsbestandteile werden nach der Sterilisation vereinigt.

50 g/l Titriplex III
22 g/l Zinksulfat x 7 $H_2O$
5.54 g/l Calciumchlorid

*
**

| | |
|---|---|
| 5.06 g/l | Manganchlorid x 4 $H_2O$ |
| 4.99 g/l | Eisensulfat x 7 $H_2O$ |
| 1.1 g/l | Ammoniumheptamolybdat x 4 $H_2O$ |
| 1.57 g/l | Kupfersulfat x 5 $H_2O$ |
| 1.61 g/l | Cobaltchlorid x 6 $H_2O$ |

mit KOH auf pH 6.0 einstellen

| | |
|---|---|
| 20 µg/l | Biotin |
| 20 000 µg/l | Ca-pantothenat |
| 2 µg/l | Folsäure |
| 10 000 µg/l | Inositol |
| 200 µg/l | p-Aminobezoesäure |
| 400 µg/l | Niacin (Nicotinsäure) |
| 400 µg/l | Pyridoxinhydrochlorid |
| 200 µg/l | Riboflavin |
| 400 µg/l | Thiaminhydrochlorid |

Vorkultur:

Für einen 10 l Fermenter wurden 2 x 250 ml Vorkultur (= VK) im 1 l Erlenmeyerkolben (= EMK) mit jeweils 3-4 Platinösen Lu 583 beimpft und 24 h bei 28°C und 200 Upm inkubiert.

Hauptkultur:

Ansatz im 10 l Infors Fermenter mit folgenden Bedingungen:

| | |
|---|---|
| Temperatur | 28°C |
| Belüftung | 5 l/min. über Ringbegasung |
| Drehzahl | 400 Upm. |
| Fermenterbestückung | 3 * Standardrührblätter sowie eingebaute Wellenbrecher |

Vor dem Start der Fermentation wurde durch die Zugabe von 50%iger (V/V) n-Butylaminlösung in Wasser der pH auf ca. 7.0 eingestellt. Anschließend wurde der Fermenter mit 2 x 250 ml Vorkultur beimpft. Die Fermentationsdauer betrug etwa 28-30 h. Der Zeitpunkt des Fermentationsende wurde über die Bestimmung der OD bei 600 nm festgelegt. Die OD 600 der Fermenterproben wurde dabei gegen Luft gemessen und sollte bei 0.9-1.0 liegen. Bei höherer OD nimmt die Aktivität des Enzyms schnell ab.

Aufarbeitung:

Der Fermenterinhalt wurde in einer Hereaus Cryofuge 8000 bei 45°C und 5000 Upm (entspricht ca. 9500 x g) 20 min. zentrifugiert. Die gesamte Biofeuchtmasse wurde in 250 ml 50 mM Kaliumphosphatpuffer gewaschen und erneut zentrifugiert. Die Zellmasse kann bei -15°C im Tiefkühlschrank gelagert werden.

Zellaufschluß und Messung der Lysyloxidase

Die Biofeuchtmasse (100 ml) wurde mit 100 ml Glasperlen (Durchmesser 0,5 mm) in der Kugelmühle unter Eiskühlung 30 Minuten bei 5 000 Umdrehungen pro Minute zerkleinert. Das Homogenat wurde über Gaze filtriert und dann für 10 Minuten bei 10 000 rpm und 4 Grad Celsius zentrifugiert. Die Aktivität des Überstandes wurde durch einen HPLC-Test ermittelt. Die Lysyloxidase wandelt Benzylamin in Benzaldehyd um (Detektion bei 250 nm). Die Menge an entstandenem Benzaldehyd wurde über eine Eichkurve ermittelt.

Laufbedingungen:

Puffer A: Wasser, 0,1 % TFA
Puffer B: Acetonitril, 0,1 % TFA

0 Minuten 40 % B
6 Minuten 70 % B
6,1 Minuten 100 % B
6,5 Minuten 100 % B
Fluß 1 ml/min
6,5 - 9 min zurück auf 40% B, Fluß 1,5 ml/min

Die Lysyloxidase enthaltenden Überstände von 7 x 10 l Fermentern wurden gesammelt und vereinigt. Es ergab sich eine Aktivität von 125,5 U/l in 1,8 l.

Beispiel 2

Markierung der Gelatine mit Fluoreszenzfarbstoffen und Quervernetzung der Gelatine durch die Lysyloxidase

Gelatine (10 g) wurde in 50 mM Natriumboratpuffer pH 9,0 gelöst. Der pH wurde kontrolliert und mit 0,5 M $NaHCO_3$ nachgestellt. Die Fluoreszenzfarbstoffe Cy-5 oder Bodipy 630/650-X, SE) wurden in 5 ml DMSO (= Dimethylsulfoxid) gelöst und in einem Gewichtsverhältnis von 1:100 den Proteinen zugesetzt. Während 16 Stunden bei Raumtemperatur reagierten die Succinimidylester der Farbstoffe mit den freien Aminogruppen der Proteine. Die Reaktionsansätze wurden dann 5 mal gegen große Volumina 20 mM Natriumphosphatpuffer mit 0,01 % Azid dialysiert und bei -20 Grad Celsius als 1%ige Proteinlösung gelagert.

In einem 4 l Rührkolben wurden in 1800 ml einer Lösung, bestehend aus vereinigten überständen von Lysyloxidase-Fermentationslösungen (ZH 29303/5; 125,5 U/l) bei 40°C 454 g Gelatine A 100 Bloom durch Rühren in Lösung gebracht. Dazu wurde mit Bodipy dotierte Gelatine (siehe oben) gegeben. Der gesamte Ansatz wurde 48 h bei 40°C gerührt, wobei der Kolben nicht geschlossen wurde, um einen dauernden Luftzutritt und damit den Zutritt von Sauerstoff zu ermöglichen.

Es wurden aus der Lösung in den angegebenen zeitlichen Abständen Proben à 300 g entnommen:

Probe 1: sofort nach Beginn des Versuchs
Probe 2: nach 2 Std.
Probe 3: nach 5 Std.
Probe 4: nach 24 Std.
Probe 5: nach 29 Std.
Probe 6: nach 48 Std.

Die Proben wurden wie folgt aufgearbeitet:

a) 200 g wurden mit einer Einstoffdüse direkt in eine Wolke aus hydrophobierter Kieselsäure (Sipernat D 17) zu Partikeln von ca. 200 µm Durchmesser versprüht. Das erhaltene feuchte Produkt wurde geteilt; eine Hälfte wurde bei Raumtemperatur und die andere bei 80°C jeweils auf einer Nutsche im Luftstrom getrocknet.

b) Die restlichen 100 g wurden nach Zusatz von Vitamin A, Isosweet und Maisstärke nach Einemulgierung der Öl-Phase mit einem Ultraturrax-Gerät in gleicher Weise versprüht und durch Trocknung des Sprühproduktes bei Raumtemperatur in ein Trockenpulver umgewandelt. Diese Produkte hatten etwa folgende Zusammensetzung:

25 % Vitamin A-Acetat stab. mit Ethoxyquin und BHT
30 % Gelatine A 100 Bloom
25 % Maisstärke
15 % Isosweet
Rest: Wasser + Sipernat D 17 + Reste aus Fermentationsrückstand

Beispiel 3

Messung der Quervernetzung durch Fluoreszenz-Korrelations-Spektroskopie (= FCS)

Die FCS ist eine Methode, die auf der Anzahlfluktuations-Spektroskopie beruht. Mit der FCS können Diffusionskoeffizienten und Anzahlkonzentrationen fluoreszierender Moleküle in hochverdünnter Lösung gemessen werden. Die Figuren 1 und 2 zeigen den Aufbau einer FCS-Apparatur und ihr Funktionsprinzip. Dabei wird ein Laserstrahl über ein Mikroskopobjektiv in ein sehr kleines Volumen einer flüssigen Probe fokussiert und die darin angeregte Fluoreszenz

gemessen.

Figur 1 zeigt eine FCS Apparatur. Der fokussierte Laserstrahl und die konfokalen Blenden definieren ein sehr kleines Beobachtungsvolumen (das konfokale Volumen) in der Probe. Die zeitlichen Schwankungen der Fluoreszenz aus diesem Volumen werden mit einem Korrelator analysiert.

Figur 2: Anzahlfluktuationen im Beobachtungsvolumen führen zu Fluktuationen des FCS-Signals I(t). Aus der Autokorrelationsfunktion G(t) des FCS-Signals können die mittlere Verweilzeit $\tau$ (= mittlere Zeit für die Diffusion eines Teilchens durch das Beobachtungsvolumen) und die Zahl der Moleküle N im Meßvolumen abgelesen werden. $\tau$ ist direkt proportional zur Größe der fluoreszierenden Moleküle.

Das Volumen ist dabei so klein, daß sich darin im Mittel nur wenige Teilchen befinden. Die Teilchenzahl im Volumen schwankt durch Diffusion ständig um einen mittleren Wert N. Damit schwankt auch die Fluoreszenzintensität I(t) um einen mittleren Wert Im. Aus den Schwankungen der Fluoreszenz lassen sich die Diffusionszeit $\tau$ (proportional zur Molekülgröße) und die Anzahlkonzentration der fluoreszierenden Moleküle berechnet (siehe Figur 1).

Bestimmung der Molekulargewichte durch Gel-Permeations-Chromatographie (GPC)

Die Bestimmung der Molekulargewichtsverteilung (MGV) erfolgte mittels GPC. Die wichtigsten Versuchsparameter:

| | |
|---|---|
| Säule: | TSK Gel, 4000 SWXL, 250 • 4 mm |
| Laufmittel: | 0,01 M NaH2PO4, 0,1 M Na2SO4, 1 % SDS |
| pH: | 5,3 |
| Temperatur: | 30°C |
| Fluß: | 0,5 ml/min |
| Detektion: | UV-Spektrometer, 220 nm |

Zur Probenvorbereitung wurden jeweils ca. 0,5 g Substanz in 100 ml Laufmittel für 15 min bei T = 60°C gerührt. Die so erhaltene trübe Lösung wurde durch einen 0,2 µm Filter tiltriert und anschließend in die Probenschleife des GPC-Gerätes injiziert (ca. 20 µl). Nicht wasserlösliche Gelatine Bestandteile konnten nicht detektiert werden.

Als direkte Meßgröße erhielt man bei dieser Methode das Intensitätssignal des Detektors (Maß für die Konzentration) als Funktion der Elutionszeit. Bei der GPC wurden die Moleküle nach ihrem hydrodynamischen Radius rH getrennt. Moleküle mit großem rH werden dabei schneller eluiert als Moleküle mit kleinem rH.

Über eine Kalibrierung mit Standard-Polymeren (hier: Pullulstandards) konnte aus den Elutionsdiagrammen MGV berechnet werden [siehe M.D. Lechner, K. Gehrke und E.H. Nordmeier, in: "Makromolekulare Chemie", Kapitel 4.3.6.1., Birkhäuser Verlag (1996)]. Zwischen der Elutionszeit tE und der Molmasse M der Pullulstandards gilt in erster Näherung folgende Proportionalität:

$$t_E \propto \log(M) \qquad (1)$$

Exakter lassen sich bei einer Auftragung von $t_E$ vs. log(M) die Daten jedoch mit einem Polynom dritten Grades beschreiben. Aus der Inversen dieses Polynoms können aus den Gelatine-Elutionszeiten Gelatine-Molekulargewichte berechnet werden. Die so erhaltenen Molekulargewichte sind somit keine absoluten Gelatine-Molekulargewichte (wie sie z.B. aus der LS erhalten werden können), sondern lediglich relative Molekulargewichte (bezogen auf die hier verwendeten Standards). Weiterhin ist zu beachten, daß aufgrund der zur Verfügung stehenden Pullulstandards die Elugramme nur im Bereich von 12 min < t < 25 min ($\equiv 10^3$ g/mol < MG < $10^7$ g/mol) sinnvoll in die entsprechenden Molekulargewichte umgerechnet werden können. Annähernd [siehe Gleichung (1)] ist bei einer halblogarithmischen Auftragung ($t_E$ vs. log(M)> die Fläche unter der Kurve der untersuchten Substanzmenge proportional. Die Elutionsdiagramme wurden auf gleiche Gesamtflächen normiert, so daß die Flächen unter den Kurven -und somit die jeweiligen Substanzmengen- direkt miteinander verglichen werden konnten.

Folgende Beobachtungen wurden gemacht: Mit zunehmender Dauer der Inkubation der markierten Gelatine mit der unmarkierten Gelatine und der Lysyloxidase wurde der Wert $\tau$ (proportional zur Molekülgröße) bei Messungen durch die FCS immer kleiner und gleichzeitig nahm die Intensität (I,kcps) der Fluoreszenz ab (Tabelle 1, Figur 3).

Weiterhin wurde in der GPC mit zunehmender Inkubationszeit eine Abnahme der hochmolekuaren Gelatine-Anteile beobachtet (Tabelle 2, Figur 4).

Durch die Inkubation der Gelatine mit Lysyloxidase wurde die Gelatine vernetzt. Diese vernetzten Anteile konnten in Wasser nicht mehr vollständig gelöst werden, sondern lagen als gequollene Gelpartikel vor. Bei der FCS-

und GPC-Probenpräparation wurden diese Partikel durch Zentrifugation oder Filtration abgetrennt und konnten nicht mehr detektiert werden.

Mit zunehmender Inkubationszeit der Gelatine verschwanden bevorzugt die hochmolekularen Gelatine-Anteile aus dem Elugramm. Dies bedeutet, daß gerade die hochmolekularen Gelatine Anteile überproportional von der Vernetzung betroffen sind. Aus statistischen Gründen ist dieses Verhalten durchaus sinnvoll. Darüberhinaus konnte aber auch eine molekulargewichtsabhängige Verteilung der Lysingruppen für diesen Befund mit verantwortlich sein.

Die Abnahme von $\tau$ durch FCS-Messungen ist als Abnahme des Molekulargewichts zu interpretieren und konnte durch Gel-PermeationsChromatographie direkt gezeigt werden. Die Abnahme der Intensität ist auf die Abnahme der Anzahl fluoreszenter Teilchen im Überstand der rückgelösten Präparate zurückzuführen. Durch Quervernetzung wurden große Gelatinemoleküle mit höherer Wahrscheinlichkeit betroffen als kleine Moleküle. Außerdem können kleine Moleküle das durch die Wirkung der Lysyloxidase entstandene Netzwerk besser verlassen als unvernetzte, große Gelatinemoleküle, die im unlöslichen Teil zurückbleiben.

Damit konnte gezeigt werden, daß durch die Lysyloxidase eine für eine Quervernetzung ausreichende Zahl von Allysinen und später Schiff'sche Basen gebildet werden.

Beispiel 4

Nachweis der Quervernetzung durch einen schnellen, parallelisierbaren Test in Mikrotiterplatten

Gelatine wurde mit 1 mM/l N-Hydroxysuccinimid aktiviertem Biotin bei pH 8,4 in einem Boratpuffer markiert und anschließend gegen einen 20 mM Phosphatpuffer pH 7,4 mehrfach dialysiert. Die Fähigkeit der so modifizierten Gelatine, Avidin oder Streptavidin zu binden, konnte in Vorversuchen gezeigt werden. 10 und 100 mg/ml Gelatine binden nach Biotin-Modifikation an die mit Avidin modifizierte Oberfläche eines Biacore-Sensorchips. Nicht modifizierte Gelatine wurde nicht an diese Oberfläche gebunden.

Testprinzip und Durchführung

Gelatine wurde an die Oberfläche von Mikrotiterplatten adsorbiert. Danach wurde Biotin-markierte Gelatine und die quervernetzende Substanz/Enzym zugefügt. Adsorbierte Gelatine wurde mit biotinylierter Gelatine vernetzt. Nach einem intensiven Waschschritt wurde die Biotinylierung durch einen StreptavidinPeroxidase-Komplex nachgewiesen.

0,4 ml einer 1%igen Gelatinelösung in 0,1 M NaHCO3, pH 9,2 wurde für 3 Stunden bei Raumtemperatur an NUNC MaxiSorp Elisaplatten adsorbiert. Danach wurde dreimal mit PBS, 0,05 % Tween 20 Lösung gewaschen.

Danach wurde biotinylierte Gelatine und in verschiedenen Konzentrationen Quervernetzer zugegeben. Dies wurde in einem PBS Puffer mit 5 mM DTT und 0,1 % Tween 20 durchgeführt. Die Mikrotiterplatten wurden anschließend für verschiedene Zeiten bei verschiedenen Temperaturen inkubiert. Danach wurden die Platte 6 mal gewaschen, um nicht-umgesetztes Material zu entfernen.

Zum Nachweis wurde Streptavidin-Peroxidase der Firma Boehringer 1:10 000 in PBS, 0,1 % Tween 20 verdünnt und 1 ml pro Well zugegeben. Es wurde erneut 30 Minuten bei Raumtemperatur (ca. 23°C) inkubiert. Nach einem weiteren Waschschritt wurden 0,015 ml Reaktionsgemisch (0,1 ml 42 mM Tetramethylbenzidin in DMSO, 10 ml 0,1 M Natriumacetat, pH 4,9 mit Zitronensäure eingestellt) zugegeben. Es bildete sich eine blaue Farblösung im Laufe der Reaktion. Die Reaktion wurde nach 5 Minuten mit 0,1 ml 2 M Schwefelsäure gestoppt. Die blaue Farbe wurde dabei gelb. Anschließend erfolgte die Messung der Absorption bei 450 nm.

Beispiel 5

Reinigung der Lysyloxidase

Homogenisation

Die Zellen von Pichia pastoris (ca 18 ml) wurden nach Lagerung bei -20 Grad C aufgetaut und mit Puffer A ( 20 mM Natriumphosphat, 1 mM Ethanolamin, pH 7,0 auf 50 ml verdünnt. Es wurden 50 ml Glasperlen, Durchmesser 0,5 mm, zugegeben und 30 Minuten bei 5000 U/ min unter Kühlung durch Eis homogenisiert. Das Homogenat wurde über Gaze filtriert. Das Filtrat wurde 10 Minuten bei 8000 rpm und 4 Grad C zentrifugiert.

Ionenaustauschchromatographie

Der Überstand wurde mit NaOH auf pH 7,0 erneut eingestellt und auf eine Q-Sepharose aufgetragen (Q-Sepharose Fast Flow, Pharmacia, Durchmesser 5 cm, Höhe 13 cm, Volumen 250 ml). Nach dem Auftrag (Leitfähigkeit 0,7 mS/cm, 540 ml) wurde mit 600 ml Puffer A gewaschen. Die Säule wurde mit einem linearem Gradient von 1 l Puffer A und 1 l Puffer B (wie Puffer A mit 1 M NaCl) eluiert. Die aktiven Fraktionen wurden gesammelt.

Molekularsiebchromatographie

Die Wertfraktion wurde nach Konzentrierung über einen 10 kDa Omega Filter auf einer präparativen Superdex-Säule (Pharmacia, Durchmesser 2,6 cm, Länge 60 cm Volumen 320 ml) in 20 mM Natriumphosphatpuffer, 150 mM NaCl, 1 mM Ethanolamin, pH 7,5 getrennt. Fluß 3 ml/Minute. Die aktiven Fraktionen wurden gesammelt.

Ionenaustauschchromatographie

Die Wertfraktion der Molekularsiebchromatographie wurde erneut durch Chromatographie an einer Mono-Q Säule gereinigt (Pharmacia, HR5/5). Der Gradient wurde aus Puffer A und Puffer B erzeugt. Flußgeschwindigkeit 1 ml/Minute, 100 Fraktionen zu je 1 ml. Die Lysyloxidase eluierte als aktives Hauptprotein. Das Protein hatte im SDS-Gel unter reduzierenden Bedingungen ein Molekulargewicht von ca 121 000 Da.

Folgende Sequenzen wurden erhalten:

Aminoterminale Sequenz

G(S)(C)Q(C)KTNEKVNIEAPKPNI(C)DT(L)(S)

Sequenzen, die Peptiden des Proteins entsprechen, die nach einem Verdau mit Trypsin erhalten wurden:

EYP(C)APGVVYNTK
GGTYSTVTQNPTLNR
DYNIMPGGGXVHR
ATGGTYSTVXAQN
APETENNAR
GPL(P)VNE(E)TTIEPLSFYNT
IYELSLQELIAEYGSDDPNNQHTFYSDI
DNVDDLSCTIIQR
VAPETENCAR
NVDVEYPCAPGVVYNTK
GYPNAEY(S)LDF/ER

Tabelle 1

| Manuelle Messungen | | | | | | |
|---|---|---|---|---|---|---|
| lfd Nr. | Zeit | Sek. | Beta | N | Tau,µs | I,kcps |
| | | | | | | |
| 66 | 0h | 61348 | 0,190 | 5,3 | 456,2 | 220,9 |
| 67 | | 61414 | 0,204 | 4,9 | 452,9 | 211,3 |
| 68 | | 61480 | 0,210 | 4,8 | 441,6 | 206,4 |
| 69 | 0h | 61562 | 0,172 | 5,8 | 455,8 | 240,3 |
| 70 | | 61628 | 0,180 | 5,6 | 450,3 | 230,1 |
| 71 | | 61696 | 0,186 | 5,4 | 456,5 | 226,6 |
| 72 | 2h | 61780 | 0,262 | 3,8 | 469,2 | 164,0 |
| 73 | | 61846 | 0,282 | 3,5 | 468,4 | 155,3 |
| 74 | | 61912 | 0,288 | 3,5 | 463,0 | 152,2 |
| 75 | 2h | 62074 | 0,314 | 3,2 | 477,0 | 139,7 |

Tabelle 1 (fortgesetzt)

| Manuelle Messungen | | | | | | |
|---|---|---|---|---|---|---|
| lfd Nr. | Zeit | Sek. | Beta | N | Tau,µs | I,kcps |
| 76 | | 62140 | 0,335 | 3,0 | 474,6 | 133,8 |
| 77 | | 62206 | 0,338 | 3,0 | 469,6 | 129,1 |
| 78 | 24h | 62354 | 0,295 | 3,4 | 345,2 | 153,4 |
| 79 | | 62420 | 0,309 | 3,2 | 358,3 | 147,8 |
| 80 | | 62486 | 0,315 | 3,2 | 346,9 | 145,6 |
| 81 | 24h | 62588 | 0,281 | 3,6 | 361,3 | 159,1 |
| 82 | | 62654 | 0,288 | 3,5 | 353,1 | 153,8 |
| 83 | | 62720 | 0,292 | 3,4 | 358,0 | 153,3 |
| 84 | 29h | 62840 | 0,324 | 3,1 | 337,0 | 138,8 |
| 85 | | 62906 | 0,335 | 3,0 | 339,7 | 133,1 |
| 86 | | 62972 | 0,340 | 2,9 | 338,0 | 131,7 |
| 87 | 29h | 63124 | 0,294 | 3,4 | 356,0 | 151,6 |
| 88 | | 63190 | 0,314 | 3,2 | 347,4 | 141,9 |
| 89 | | 63256 | 0,325 | 3,1 | 345,1 | 139,5 |
| 90 | 48h | 63334 | 0,559 | 1,8 | 288,2 | 84,7 |
| 91 | | 63400 | 0,574 | 1,7 | 292,5 | 83,6 |
| 92 | | 63466 | 0,570 | 1,8 | 288,1 | 83,9 |
| 93 | 48h | 63544 | 0,441 | 2,3 | 292,8 | 104,4 |
| 94 | | 63610 | 0,458 | 2,2 | 299,6 | 102,1 |
| 95 | | 63676 | 0,460 | 2,2 | 296,5 | 100,2 |
| | | | | | | |
| 96 | Bodipy | 63750 | 4,292 | 0,2 | 99,5 | 9,5 |
| 97 | | 63816 | 4,669 | 0,2 | 98,5 | 8,9 |
| 98 | | 63882 | 4,582 | 0,2 | 102,1 | 8,8 |

Tabelle 2

| Zeit | Probenbezeichnung | Mn [g/mol] | Mw [g/mol] | Polyd. (D) |
|---|---|---|---|---|
| | | | | |
| 0 h | 63/192-1 | 35000 | 149000 | 4,26 |
| 2 h | 63/192-2 | 35000 | 145000 | 4,13 |
| 24 h | 63/192-4 | 21000 | 39000 | 1,86 |
| 29 h | 63/192-5 | 20000 | 35000 | 1,76 |
| 48 h | 63/192-6 | 17000 | 28000 | 1,66 |

**Patentansprüche**

**1.** Verfahren zur Herstellung von Wirkstoffzubereitungen, in denen ein oder mehrere Wirkstoffe von mindestens einer

Schicht umgeben sind, dadurch gekennzeichnet, daß mindestens eine der den oder die Wirkstoffe umgebenden Schichten oder Teile dieser Schichten aus einem Protein bestehen, das mit einem Enzym ausgewählt aus der Gruppe der Lipoxygenasen, Proteindisulfidisomerasen, Phenoloxidasen und Peroxidasen, Lysyloxidasen, Proteindisulfidreduktasen, Tyrosinoxidasen oder Sulfhydryloxidasen quervernetzt wurde.

**2.** Verfahren zur Herstellung von Wirkstoffzubereitungen nach Anspruch 1, dadurch gekennzeichnet, daß der oder die Wirkstoffe oder ein oder mehrere mit mindestens einer Schicht umgebende Wirkstoffe in Lösung mit vernetzbarem Protein und Enzym zur Beschichtung gemischt wird, wobei das Gewichtsverhältnis zwischen Wirkstoff und Protein zwischen 1 zu 100 und 5 zu 1 liegt.

**3.** Verfahren nach Anspruch 1 oder 2, dadurch gekennzeichnet, daß als Wirkstoff Vitamine, Enzyme, Lebensmittelzusatzstoffe oder Futtermittelzusatzstoffe verwendet werden.

**4.** Verfahren nach den Ansprüchen 1 bis 3, dadurch gekennzeichnet, daß der Wirkstoff hydrophob ist.

**5.** Verfahren nach den Ansprüchen 1 bis 4, dadurch gekennzeichnet, daß ein vernetzbares Protein ausgewählt aus der Gruppe Gelatine, Kasein, Sojaprotein, Weizenprotein, Maisprotein oder Kollagen verwendet wird.

**6.** Verfahren nach den Ansprüchen 1 bis 5, dadurch gekennzeichnet, daß ein aus Mikroorganismen gewonnenes Enzym verwendet wird.

**7.** Verfahren nach den Ansprüchen 1 bis 6, dadurch gekennzeichnet, daß die Quervernetzung mit dem Enzym Lysyloxidase durchgeführt wird.

**8.** Verfahren nach den Ansprüchen 1 bis 7, dadurch gekennzeichnet, daß man mindestens einen Wirkstoff mit einer wäßrigen Lösung aus vernetzbarem Protein und Enzym durchmischt und versprüht.

**9.** Verfahren nach Anspruch 8, dadurch gekennzeichnet, daß in einer mit hydrophober Kieselsäure, Maisstärke oder hydrophobierte Maisstärke beladenen Atmosphäre versprüht wird.

**10.** Verfahren nach den Ansprüchen 1 bis 9, dadurch gekennzeichnet, daß die Wirkstoffzubereitung bis zu einer Restfeuchte unter 10 Gew.-% getrocknet wird.

**11.** Verfahren nach den Ansprüchen 1 bis 10, dadurch gekennzeichnet, daß die Temperatur bei der Herstellung der Wirkstoffzubereitung im Wesentlichen unter 80°C gehalten wird.

**12.** Wirkstoffzubereitung erhältlich nach einem Verfahren gemäß den Ansprüchen 1 bis 11.

**13.** Wirkstoffzubereitung enthaltend mindestens eine einen oder mehrere Wirkstoffe umgebende Schicht oder Teile einer Schicht aus einem Protein, das mit einem Enzym ausgewählt aus der Gruppe der Lipoxygenasen, Proteindisulfidisomerasen, Phenoloxidasen und Peroxidasen, Lysyloxidasen, Proteindisulfidreduktasen, Tyrosinoxidasen oder Sulfhydryloxidasen quervernetzt wurde.

**14.** Wirkstoffzubereitung nach Anspruch 13, wobei die Wirkstoffe ausgewählt sind aus der Gruppe:

Carotinoide
Xanthophylle
Vitamin A
Vitamin E
Vitamin $D_3$
Vitamin $K_1$

**15.** Wirkstoffzubereitung nach Anspruch 13 oder 14, die den 0,025-fachen bis 4-fachen Gewichtsanteil bezüglich Wirkstoff an Trennmittel oder Trennmittelgemischen enthält.

**16.** Wirkstoffzubereitung nach den Ansprüchen 13 bis 15, wobei der Wirkstoffgehalt in der Wirkstoffzubereitung zwischen 1 bis 75 Gew.-% liegt.

**17.** Ein isoliertes Protein, das mindestens eine der folgenden Aminosäuresequenzen enthält

G(S)(C)Q(C)KTNEKVNIEAPKPNI(C)DT(L)(S)
EYP(C)APGVVYNTK
GGTYSTVTQNPTLNR
DYNIMPGGGXVHR
ATGGTYSTVXAQN
APETENNAR
GPL(P)VNE(E)TTIEPLSFYNT
IYELSLQELIAEYGSDDPNNQHTFYSDI
DNVDDLSCTIIQR
VAPETENCAR
NVDVEYPCAPGVVYNTK
GYPNAEY(S)LDF/ER

und das die ε-Aminogruppen des Lysins zu Aldehydgruppen oxidieren kann.

**18.** Verwendung eines Enzyms ausgewählt aus der Gruppe der Lipoxygenasen, Proteindisulfidisomerasen, Phenoloxidasen und Peroxidasen, Lysyloxidasen, Proteindisulfidreduktasen, Tyrosinoxidasen oder Sulfhydryloxidasen zur Formulierung von Wirkstoffzubereitungen.

**19.** Lebensmittel oder Futtermittel, enthaltend eine Wirkstoffzubereitung nach den Ansprüchen 13 bis 16.

**20.** Pharmazeutische Zubereitung, enthaltend eine Wirkstoff-zubereitung nach den Ansprüchen 13 bis 16.

FIG.1

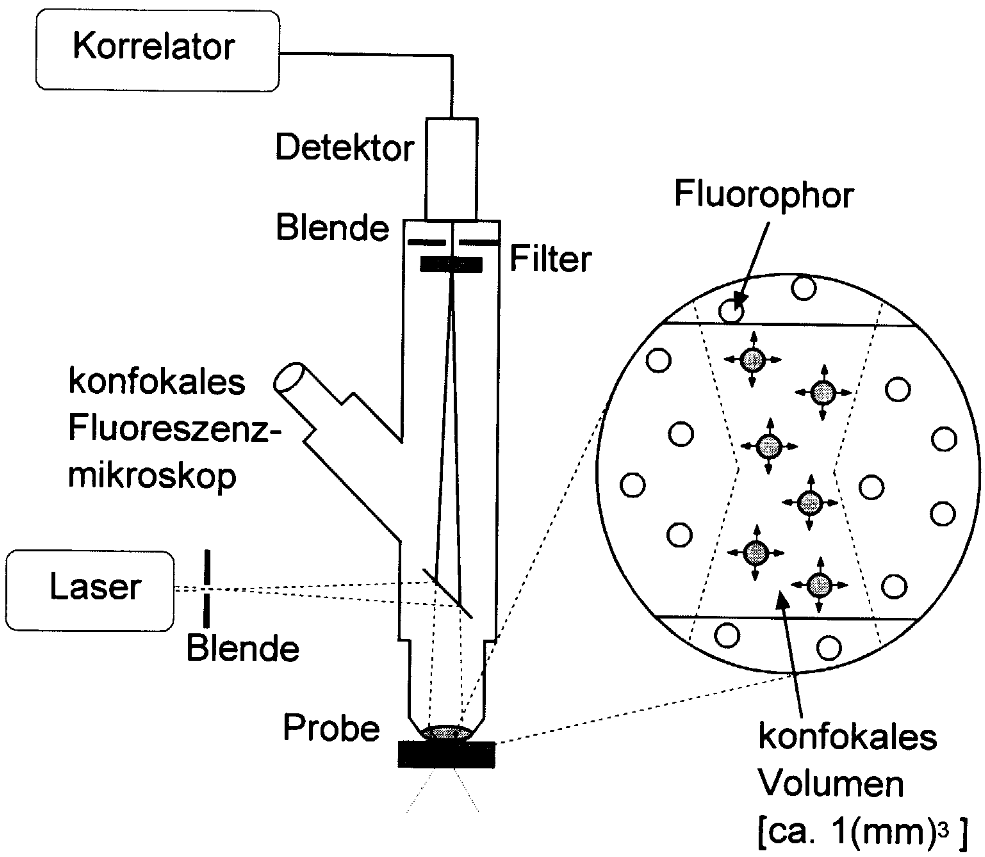
Korrelator
Detektor
Blende
Filter
Fluorophor
konfokales
Fluoreszenz-
mikroskop
Laser
Blende
Probe
konfokales
Volumen
[ca. 1(mm)3 ]

FIG.2

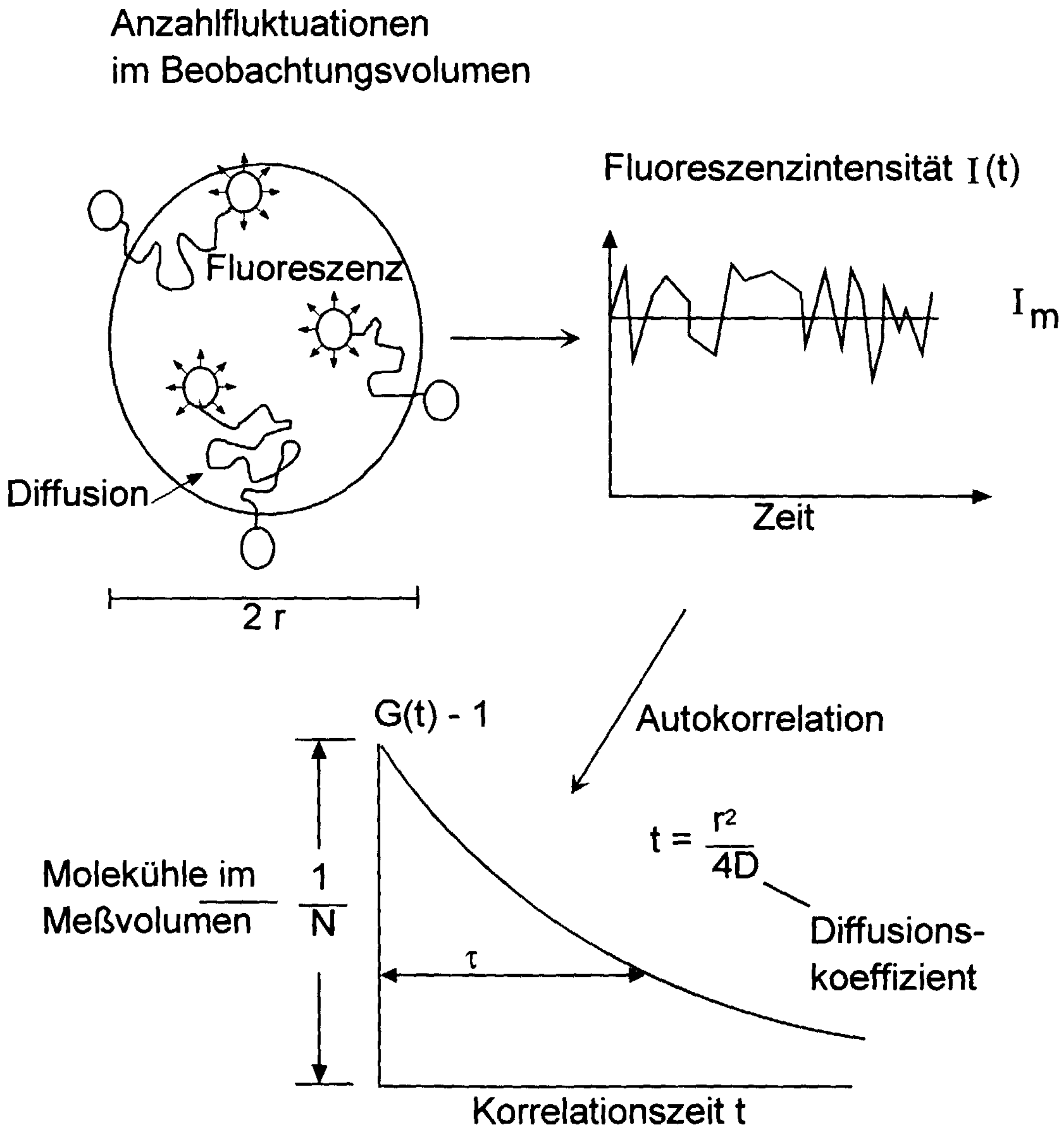
Anzahlfluktuationen
im Beobachtungsvolumen
Fluoreszenz
Diffusion
2 r
Fluoreszenzintensität I(t)
I m
Zeit
Autokorrelation
G(t) - 1
Molekühle im Meßvolumen
1/N
τ
t = r²/4D
Diffusions-koeffizient
Korrelationszeit t

FIG.3

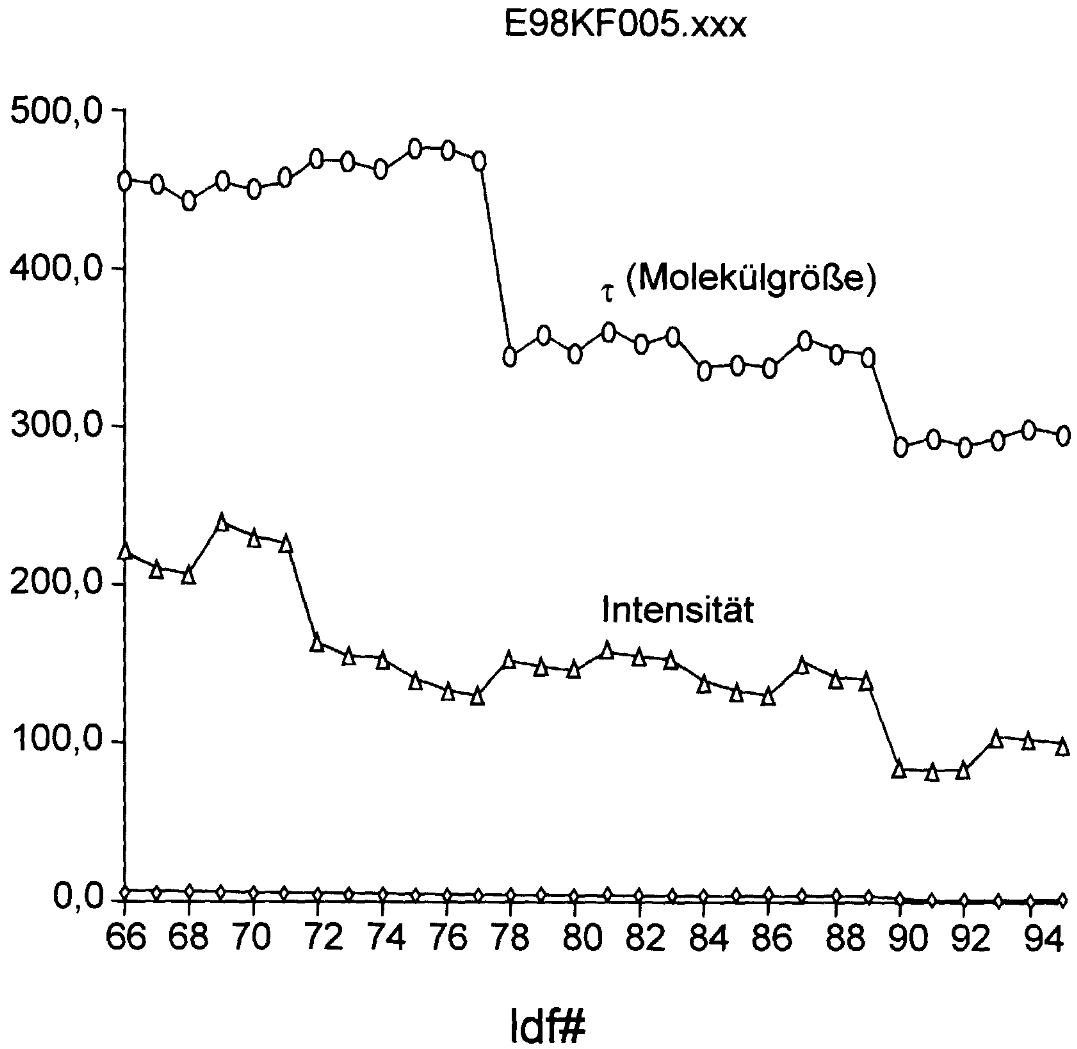
E98KF005.xxx
500,0
400,0
300,0
200,0
100,0
0,0
τ (Molekülgröße)
Intensität
66 68 70 72 74 76 78 80 82 84 86 88 90 92 94
ldf#

FIG.4

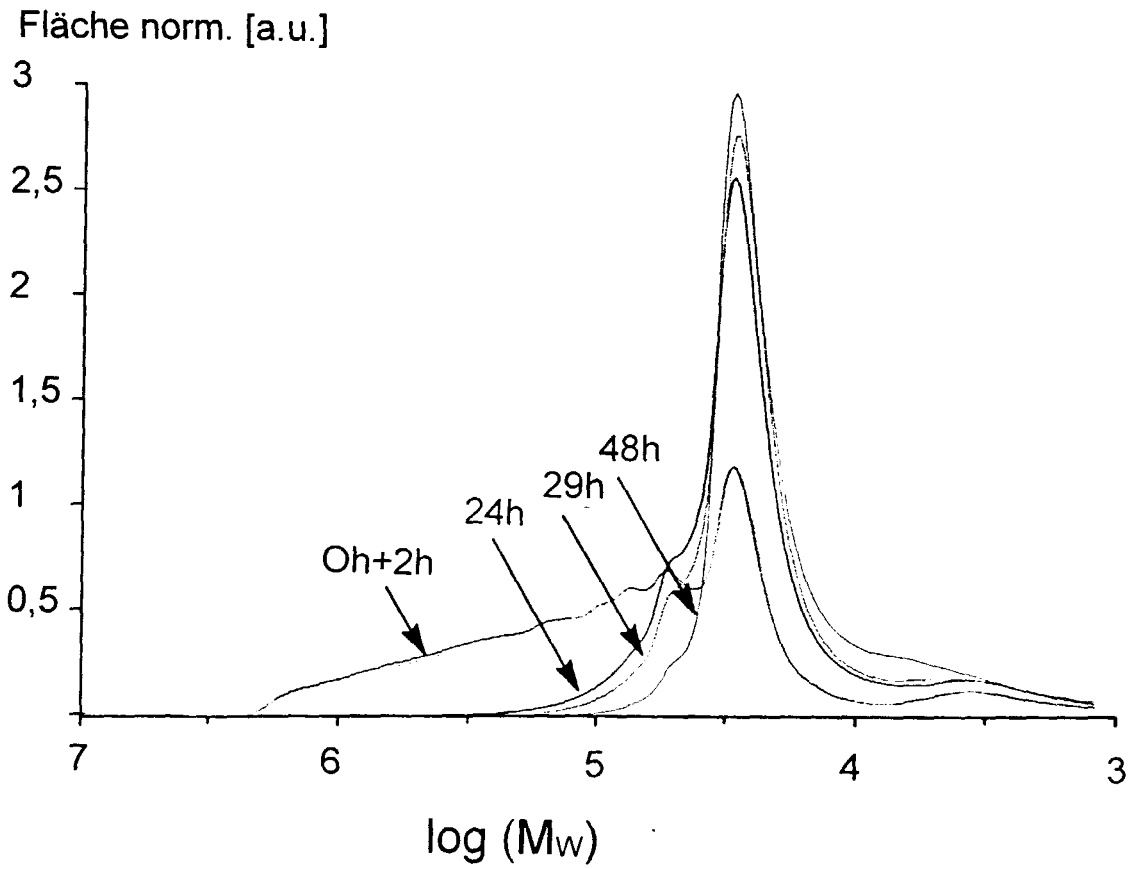

Europäisches Patentamt

# EUROPÄISCHER RECHERCHENBERICHT

Nummer der Anmeldung
EP 99117095.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 7) |
|---|---|---|---|
| Y | EP 0856355 A (GIVAUDAN-ROURE (INTERNATIONAL) S.A.) 05 August 1998, Zusammenfassung, Ansprüche. -- | 1-3, 5-7, 11-13, 18-20 | A61K9/38 A23J3/00 B01J13/14 C08J5/18 C08L89/00 |
| Y | WO 97/40701 A (ZYMOGENETICS, INC.) 06 November 1997, Zusammenfassung, Seite 17, Zeilen 7-13,33-36, Seite 18, Zeilen 1-17, Ansprüche. -- | 1-3, 5-7, 12,13, 19,20 | |
| Y | PATENT ABSTRACTS OF JAPAN, Band 96, Nr. 1, 31 Januar 1996; & JP 07 247373 A (AJINOMOTO TAKARA CORP.KK.)) 26 September 1995. -- | 1,5-7, 11,18 | RECHERCHIERTE SACHGEBIETE (Int. Cl 7) |
| Y | JOURNAL OF FOOD BIOCHEMISTRY, Band 11, Nr. 1, 29 Mai 1987, Seiten 309-327, MATHEIS G. ET AL:"A REVIEW: ENZYMATIC CROSS_LINKING OF PROTEINS APPLICABLE TO FOODS", Zusammenfassung, Seite 310 (in der Beschreibung genannt). ---- | 13,18, 19 | A61K A23J B01J C08J C08L |

Der vorliegende Recherchenbericht wurde für alle Patentansprüche erstellt.

| Recherchenort | Abschlußdatum der Recherche | Prüfer |
|---|---|---|
| WIEN | 09-12-1999 | KRENN |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

ANHANG ZUM EUROPÄISCHEN RECHERCHENBERICHT
ÜBER DIE EUROPÄISCHE PATENTANMELDUNG NR. EP 99117095.2

In diesem Anhang sind die Mitglieder der Patentfamilien der im obengenannten europäischen Recherchenbericht angeführten Patentdokumente angegeben.
Die Angaben über die Familienmitglieder entsprechen dem Stand der EPIDOS-INPADOC-Datei am 15.12.1999
Diese Angaben dienen zur Unterrichtung und erfolgen ohne Gewähr.

| Im Recherchenbericht angeführtes Patentdokument | | Datum der Veröffentlichung | Mitglied(er) der Patentfamilie | | Datum der Veröffentlichung |
|---|---|---|---|---|---|
| EP A2 | 856355 | 05-08-1998 | AU A1 | 52841/98 | 06-08-1998 |
| | | | BR A | 9800513 | 25-05-1999 |
| | | | CA AA | 2228165 | 31-07-1998 |
| | | | EP A3 | 856355 | 28-10-1998 |
| | | | JP A2 | 10249184 | 22-09-1998 |
| | | | ZA A | 9800585 | 31-07-1998 |
| WO A1 | 9740701 | 06-11-1997 | AU A1 | 29240/97 | 19-11-1997 |
| | | | US A | 5834232 | 10-11-1998 |
| JP A2 | 7247373 | 26-09-1995 | keine | | |

Bezüglich näherer Einzelheiten zu diesem Anhang siehe Amtsblatt des Europäischen Patentamtes, Nr. 12/82.